Europäisches Patentamt

European Patent Office

Office européen des brevets

(19)

(11) Numéro de publication : **0 109 935**
**B1**

(12) # FASCICULE DE BREVET EUROPÉEN

(45) Date de publication du fascicule du brevet :
01.02.89

(21) Numéro de dépôt : **83810524.5**

(22) Date de dépôt : **14.11.83**

(51) Int. Cl.⁴ : **A 61 N 5/06**, A 61 N 1/04,
A 01 N 1/00

(54) Appareil de traitement de tissus vivants par stimulation au moyen d'impulsions de courant électrique et/ou d'ondes électromagnétiques.

(30) Priorité : **15.11.82 CH 6637/82**

(43) Date de publication de la demande :
**30.05.84 Bulletin 84/22**

(45) Mention de la délivrance du brevet :
**01.02.89 Bulletin 89/05**

(84) Etats contractants désignés :
**AT CH DE LI NL SE**

(56) Documents cités :
CH--A-- 117 891
DE--A-- 3 023 130
DE--C-- 121 176
FR--A-- 631 893
FR--A-- 644 157
FR--A-- 2 066 680
FR--A-- 2 207 733
FR--A-- 2 329 258
FR--A-- 2 391 738
US--A-- 2 884 926
US--A-- 3 749 100
US--A-- 4 153 060
US--A-- 4 273 109
US--A-- 4 305 390
LASER UND ELEKTRO-OPTIK, no. 1, 1981, AT-Fachverlag, Stuttgart (DE). L. HAZYAY et al.: "Die Behandlung des Ulcus simplex vesicae urinariae mit Laserstrahlen", page 34

(73) Titulaire : **SYMTONIC S.A.**
**5, Avenue des Jordils**
**CH-1006 Lausanne (CH)**

(72) Inventeur : **Charmillot, René**
**Chemin des Pinsons 17**
**CH-2800 Delemont (CH)**
Inventeur : **Lebet, Jean-Pierre**
**Collondales 38**
**CH-1820 Montreux (CH)**

(74) Mandataire : **Patentanwälte Müller-Boré, Deufel,**
**Schön, Hertel, Lewald, Otto**
**Isartorplatz 6**
**D-8000 München 2 (DE)**

Jouve, 18, rue St-Denis, 75001 Paris, France

## Description

La présente invention concerne un appareil pour le traitement thérapeutique de tissus vivants par stimulation au moyen d'impulsions de courant électrique et/ou d'ondes électromagnétiques, comprenant un générateur d'énergie électrique.

Un appareil de ce genre est connu de FR-A-2 066 680. Ce dispositif est conçu pour le traitement des affections du système nerveux et des maladies mentales et somatiques. A ce propos, des impulsions lumineuses et des oscillations acoustiques agissent sur le système nerveux central du sujet et une source de champ électromagnétique à fréquence ultra-haute est placée au voisinage des ganglions nerveux du sujet. Un bloc de commande raccordé électriquement aux sources de radiation lumineuse, d'oscillations acoustiques et de champ électromagnétique à fréquence ultra-haute et commandant ces dernières de façon à assurer l'envoi simultané des impulsions lumineuses, acoustiques, et des impulsions du champ électromagnétique à fréquence ultra-haute est prévu.

On a déjà proposé l'utilisation d'appareil permettant de maintenir le potentiel négatif de cellules humaines, animales et végétales et/ou de faire pénétrer des substances dans les cellules (FR-A-2 391 738). Une tête de traitement à haute fréquence est rapprochée de la partie du tissu et a pour effet que le transport d'ions réalisé par deux électrodes d'ionisation rencontre une résistance dans le·tissu.

FR-A-644 157 décrit un dispositif pour le traitement des muqueuses par exemple du nez, des oreilles, du rectum, afin de prévenir ou de guérir les catarrhes, les tumeurs, etc. A ce propos un ou plusieurs corps chauffants électriques à basse tension avec une disposition permettant de maintenir ces corps chauffants dans une cavité du corps sont utilisés.

La présente invention a pour but de fournir un appareil permettant la mise en œuvre d'un genre de traitement nouveau, combinant une pluralité de stimulations par des courants électriques ou des ondes électromagnétiques, ces stimulations étant appliquées successivement ou simultanément en une même région d'un organisme vivant.

A cet effet, l'appareil selon l'invention est caractérisé par le fait que le moyen pour appliquer l'énergie électrique au tissu à traiter est un applicateur endobuccal ou endonasal adapté pour transmettre l'énergie électrique reçue du générateur, que le générateur est adapté pour générer des impulsions de courant continu de forme rectangulaire, dont la durée d'impulsion est variable de 0,5 à 5 millisecondes, dont l'amplitude est variable de 10 à 100 millivolts et dont la fréquence de répétition est variable de 10 à 100 hertz ou le générateur est adapté pour générer des impulsions de courant alternatif, dont la fréquence est variable de 20 à 100 megahertz, modulé avec une fréquence de modulation variable de 2,5 à 6 000 hertz.

D'autres caractères de l'invention sont contenues dans les sous-revendications.

L'invention sera mieux comprise en se référant au dessin annexé, dans lequel :

La figure 1 est un schéma d'ensemble de l'appareil conformément à une première forme d'exécution ;

La figure 2 représente une forme d'exécution particulière d'une sonde endonasale pouvant faire partie de l'appareil représenté à la figure 1 ;

La figure 3 est une vue en coupe, à échelle agrandie par rapport à celle de la figure 2, de la partie avant de la sonde représentée à la figure 2 ; et

La figure 4 est une vue en coupe, à même échelle que celle de la figure 3, de la partie arrière de la sonde représentée à la figure 2 ;

La figure 5 représente une autre forme d'exécution d'une sonde endonasale

La figure 6 est une vue en coupe d'une sonde endonasale selon une variante de la forme d'exécution illustrée à la figure 5 ;

La figure 7 représente un applicateur d'énergie, en forme de fourche, pouvant être utilisé en remplacement des sondes endonasales illustrées aux figures 2 à 6 ;

La figure 8 est une vue en coupe de l'applicateur illustré à la figure 7, selon le plan indiqué par la ligne VIII-VIII de la figure 7 ;

Les figures 8 (b) à 8 (d) sont des vues de coupe, analogues à la figure 8, illustrant des variantes de forme des applicateurs d'énergie représentés aux figures 7, 7 (b) et 8 ;

La figure 9 est une vue partielle de l'applicateur de la figure 7, selon le plan IX-IX de la figure 7 ;

La figure 10 est une vue partielle de l'applicateur de la figure 7, selon le plan X-X de la figure 7 ;

La figure 11 est une vue partielle de l'applicateur de la figure 7, selon le plan XI-XI de la figure 7 ;

Les figures 12 (a) à 12 (c) représentent, respectivement, une vue en élévation latérale et une vue de dessus d'un support pour deux sondes endonasales ainsi qu'une vue schématique illustrant l'utilisation de ce support ; et

Les figures 13 (a) à 13 (c) sont des vues schématiques illustrant l'utilisation d'un applicateur d'énergie endobuccal, pouvant être utilisé dans l'appareil selon l'invention.

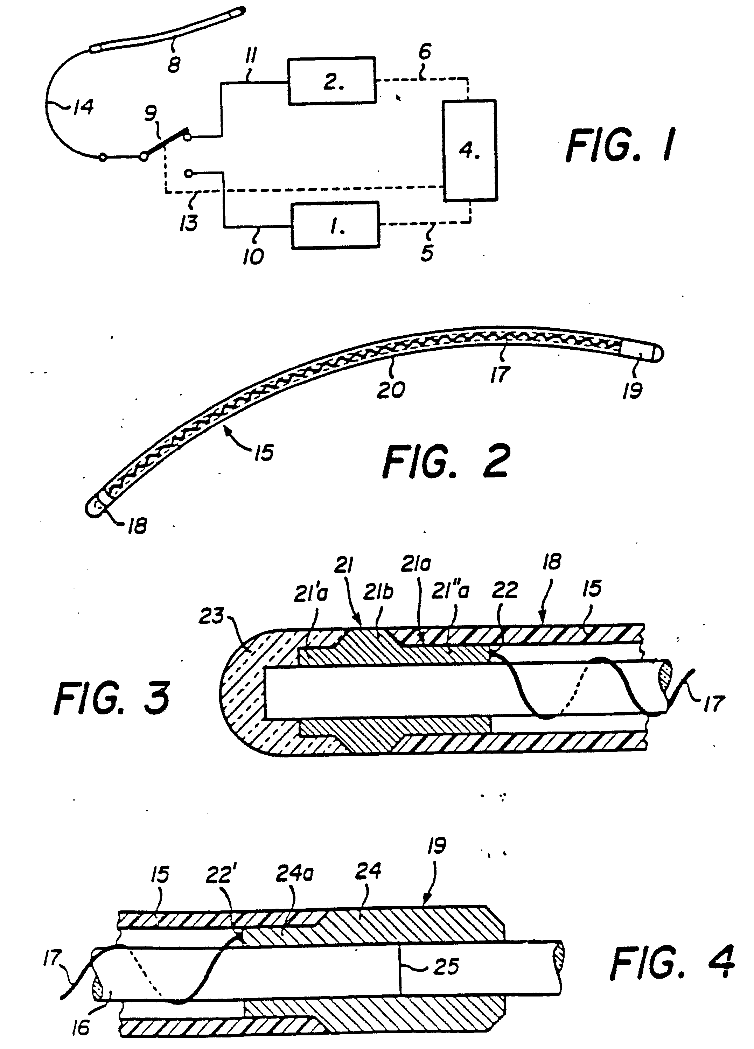

L'appareil représenté à la figure 1 comprend un générateur d'impulsions à basse fréquence de courant électrique continu 1, capable de produire des impulsions de courant sous forme d'un signal rectangulaire ayant une largeur d'impulsions de l'ordre de 0,5 à 5 millisecondes, avec une fréquence de 10 à 100 Hertz, sous une tension de 10 à 100 millivolts, et un générateur de courant alternatif à haute fréquence, modulé à basse fréquence 2, capable de produire un signal électrique formé d'une onde sinusoïdale ayant une fréquence de 20 à 100 Mégahertz, modulée sous forme sinusoïdale avec une fréquence de modulation de 2,5 à 6 000 Hertz, la puissance de ce générateur 2 étant, de préférence, de l'ordre de 0,1 à 1 Watt.

Un dispositif de commande et d'affichage 4, relié au générateur 1 et 2 par les lignes de transmission de signaux de commande respectives 5 et 6, permet de commander l'émission d'énergie par ces générateurs selon les besoins du traitement que l'on désire effectuer au moyen de l'appareil, conformément à un programme pré-établi dans lequel on fait varier les caractéristiques des signaux électriques ainsi que la durée d'émission et la séquence d'application de chacun des signaux, et d'afficher les données du programme ainsi choisi.

Les générateurs 1 et 2 sont respectivement reliés à une sonde d'application d'énergie 8, par l'intermédiaire des lignes de transmission de signaux électriques 10 et 11, et d'un commutateur 9, également commandé par le dispositif de commande et d'affichage 4, par l'intermédiaire d'une ligne de transmission de signaux de commande 13.

Le commutateur 9 est agencé de manière à permettre de relier successivement ou simultanément les lignes de transmission de signaux 10 et 11 à la sonde d'application d'énergie 8, la liaison entre la sonde 8 et le commutateur 9 s'effectuant au moyen d'une ligne de transmission de signaux électriques. Le retour du courant électrique s'effectue au moyen d'une poignée conductrice électrique, non représentée, tenue à la main par le patient pendant.l'application des signaux électriques, et reliée au générateur 1 et 2 par une ligne conductrice électrique également non représentée.

La sonde endonasale 8, représentée à la figure 2, comprend une partie médiane 20 constituée par une gaine isolante électrique 15, souple ou semi-rigide, par exemple en matière plastique, dans laquelle est en spirale une ligne de transmission de signaux électriques 17, une partie avant 18 destinée à être placée à l'intérieur de la cavité endonasale d'un patient et une partie arrière 19 destinée à permettre le raccordement avec la ligne de transmission 14.

La partie avant 18 de la sonde 8, représentée à la figure 3, comprend une pièce tubulaire 21 en un matériau bon conducteur électrique, par exemple en cuivre ou en laiton, formé d'une partie cylindrique allongée 21a, entourée d'une partie annulaire 21b, ces deux parties pouvant être d'un seul tenant, comme représenté à la figure 3, mais pouvant également être constituées par deux pièces distinctes assemblées entre elles par tout moyen approprié, par exemple par brasage, permettant le passage aisé de l'électricité d'une partie à l'autre. La partie annulaire 21b est placée légèrement plus près de l'extrémité de la partie allongée 21a destinée à être placée du côté de l'avant de la sonde 8, de sorte que la longueur de la section 21'a tubulaire de la partie 21a dépassant vers l'avant est inférieure à celle de la partie 21"a, qui dépasse vers l'arrière. Cette dernière section tubulaire 21"a, qui dépasse force dans la gaine 15 de manière à assurer la solidarité de la pièce 21 avec cette gaine 15.

L'extrémité de la ligne de transmission de signaux électriques 17, constituée, par exemple, par un fil de cuivre, est reliée électriquement à la pièce 21, par exemple au moyen d'un point de soudure 22.

Un embout arrondi 23 est fixé sur la section tubulaire 21'a en entourant cette section de sorte que la paroi latérale de la partie annulaire 21b de la pièce 21 est dénudée et affleure entre la paroi extérieure de la gaine 15 et la surface de l'embout 23, en formant une surface annulaire conductrice de l'électricité. Avantageusement, la paroi latérale de la partie annulaire 21b restant ainsi à nu est recouverte d'un revêtement bon conducteur électrique résistant à l'action des milieux physiologiques, par exemple d'une mince couche d'or.

La partie arrière 19 de la sonde 8 comprend une pièce tubulaire 24 en un matériau bon conducteur électrique, par exemple en cuivre ou en laiton. Une partie cylindrique allongée 24a est enfoncée de force dans la gaine 15 de la même façon et dans le même but que la section 21"a de la partie tubulaire 21a de la pièce 21 enfoncée à l'autre extrémité de la gaine 15. L'extrémité arrière du fil conducteur 17 est reliée électriquement à la pièce 24 par un point de soudure 22'.

On comprend que la partie avant 18 de la sonde 8 permet d'appliquer de l'énergie sous forme de courant électrique de l'organisme à traiter, en l'occurrence sur la muqueuse endonasale.

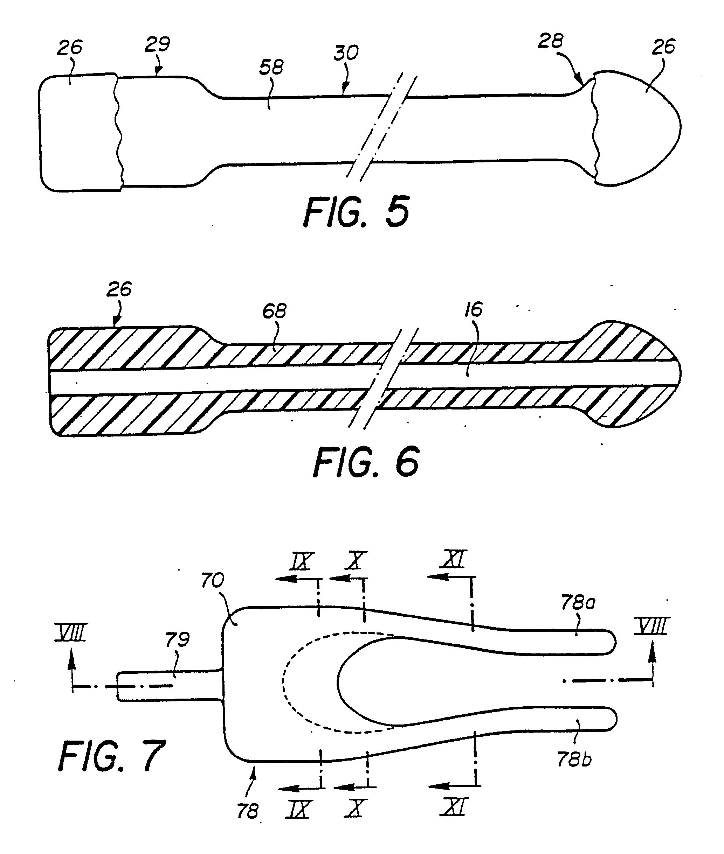

La sonde endonasale représentée à la figure 5 a la forme générale d'un stylet ou, plus précisément, d'un cathéter. Elle se compose d'une partie médiane 30 rectiligne, allongée, dont seules les extrémités sont montrées à la figure 5, pour en faciliter la représentation, ainsi que d'une partie avant 28 de forme arrondie afin de faciliter l'introduction de la sonde dans le cavum endonasal sans risque de blessure ou d'irritation de la muqueuse, et d'une partie arrière 29 de forme cylindrique. Comme on le voit à la figure 5, les parties avant 28 et arrière 29 ont un diamètre légèrement supérieur à celui de la partie médiane 30, l'échelle de la figure 5 correspondant approximativement à un facteur d'agrandissement de 10 par rapport aux dimensions réelles. Le corps 58 de la sonde représenté à la figure 5 peut être, par exemple, constitué par une seule pièce venue de moulage en une matière plastique ou élastomère, synthétique ou naturelle telle qu'une résine synthétique comme l'ABS, ou le caoutchouc dur. Avantageusement, ce corps 58 présente une certaine flexibilité afin de faciliter l'adaptation de sa forme à celle des cavités endonasales et de contribuer à la réduction du risque de blessure de la muqueuse lors de la mise en place de la sonde dans ces cavités.

La surface extérieure du corps 58 est recouverte par une couche 26 de matière conductrice électrique, constituée, par exemple, par une couche métallique mince formée pour toute technique connue appropriée, telle que la métallisation sous vide, la pulvérisation thermique, etc. Cette couche conductrice 26 a pour fonction de permettre la transmission du courant électrique et/ou des ondes électromagnétiques provenant des générateurs d'énergie de l'appareil selon l'invention, de la partie arrière 29 de la sonde, qui permet le raccordement avec la ligne de transmission d'énergie provenant de ces générateurs, jusqu'à la partie avant 28 qui constitue l'organe applicateur de courant ou des ondes dans la région bien localisée de la surface de la muqueuse endonasale avec laquelle elle est en contact. Avantageusement, cette couche conductrice 26 est constituée par un matériau ayant une résistance élevée à l'oxydation et à l'action des milieux physiologiques, par exemple l'or, l'argent, l'aluminium, le chrome, etc. Le recouvrement de la surface du corps 58 par la couche 26 peut être total ou seulement partiel, par exemple sous forme de bandes continues recouvrant la partie médiane 30 et reliant des zones de surfaces plus étendues recouvrant entièrement la partie avant 28 et la partie arrière 29 de la sonde. La partie arrière 29 joue le rôle de pièce de connexion analogue à la partie arrière 19 de la sonde 8 de la forme d'exécution illustré aux figures 2 à 4.

Il est à remarquer que la couche conductrice 26 est représentée à la figure 5 avec une épaisseur exagérée par rapport aux proportions réelles entre cette épaisseur et le diamètre des différentes parties de la sonde, afin de faciliter la compréhension de l'agencement de la sonde.

L'applicateur d'énergie 78 représenté aux figures 7 à 11 a la forme générale d'une fourche composée d'une partie médiane 70, de deux branches recourbées 78a et 78b et d'une tige de connexion cylindrique 79 permettant le raccordement électrique de l'applicateur aux générateurs d'impulsions de courant électrique et/ou d'ondes électromagnétiques.

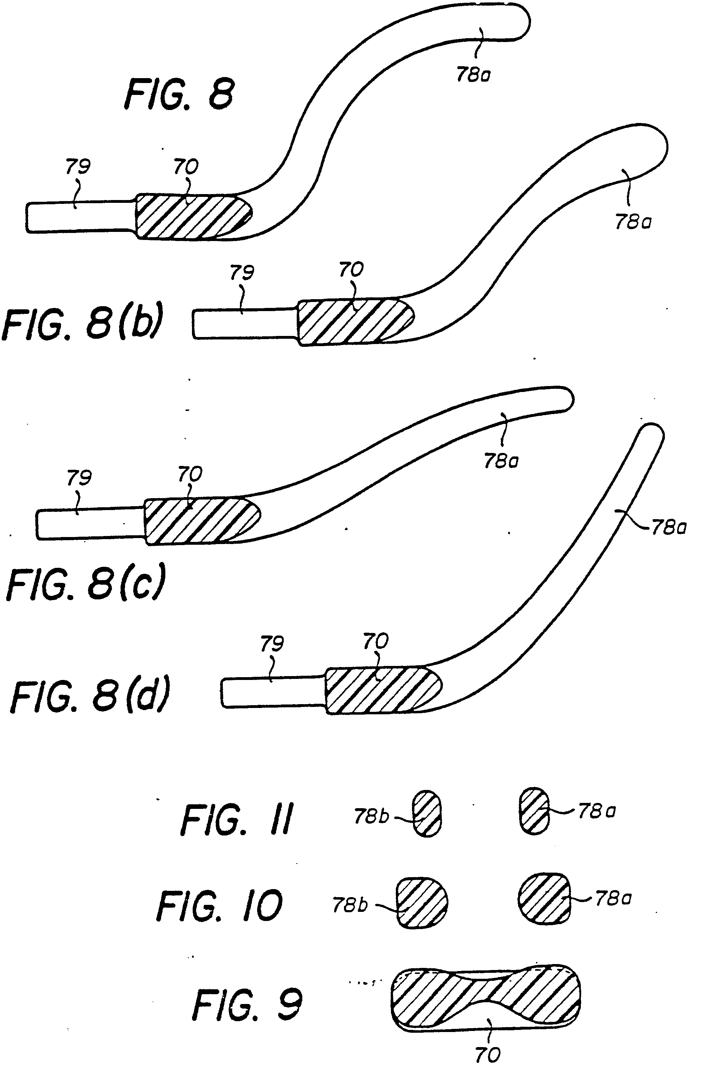

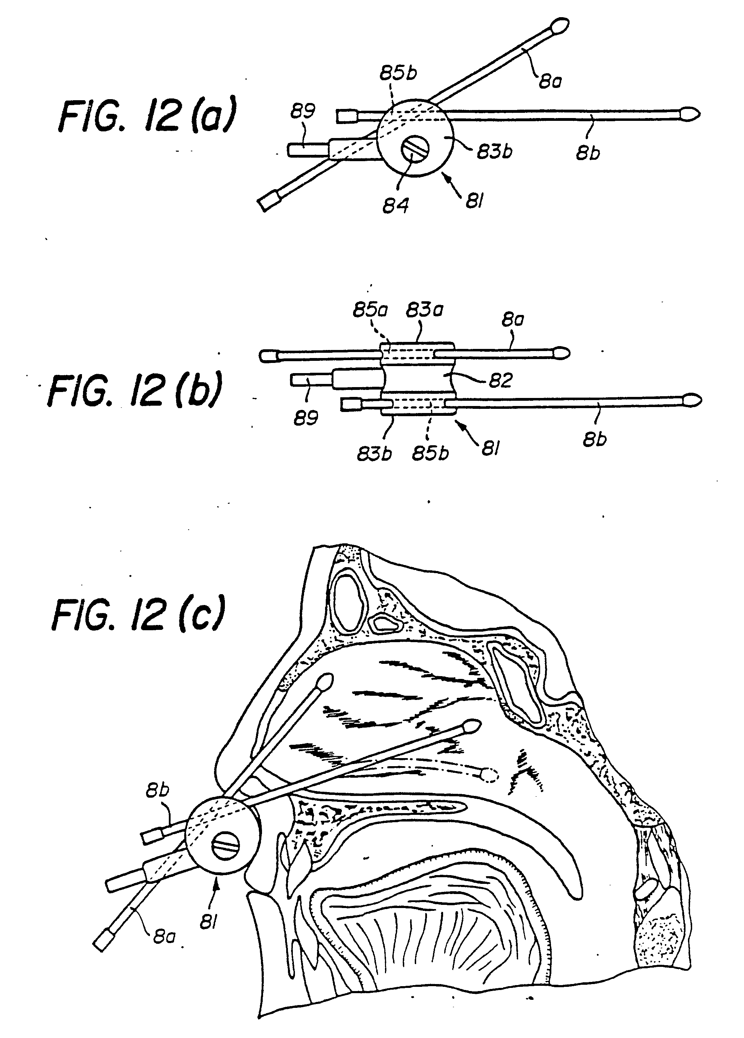

Les vues en coupe de l'ensemble de l'applicateur 78 (figure 8) et de différentes parties de celui-ci, à savoir de la partie médiane 70 (figure 9), et de deux sections des branches 78a et 78b (figures 10 et 11) montrent les détails de la forme de cet applicateur.

Mise à part sa forme particulière, qui a pour but de garantir à l'utilisateur une grande sécurité en limitant les risques de blessure et d'irritation de la muqueuse endonasale, l'applicateur 78 a un agencement et un fonctionnement similaires à ceux de la sonde illustrée à la figure 5. En particulier, il peut être constitué par une seule pièce venue de moulage, en matière plastique, entièrement ou partiellement recouverte d'une couche conductrice électrique (non représentée) ayant la même fonction que la couche 26 de la sonde de la figure 5.

De nombreuses variations peuvent être apportées, comme illustré, par exemple, aux figures 8(b), 8(c) et 8(d), à la forme de l'applicateur 78, notamment en ce qui concerne l'écartement, l'inclinaison et les rayons de courbure des branches 78a et 78b. Les dimensions de l'applicateur peuvent également varier, notamment pour permettre l'adaptation aux dimensions des cavités nasales des utilisateurs, ainsi qu'aux différents endroits précis d'application de l'énergie.

Le support 81 représenté aux figures 12(a), 12(b) et 12(c) se compose d'une partie centrale 82 et de deux parties latérales 83a et 83b disposées de part et d'autre de la partie centrale 82 et mobiles en rotation par rapport à celle-ci, autour d'un axe constitué par une vis 84.

Les trois parties 82, 83a et 83b du support 81 sont, de préférence, réalisées sous forme de pièces moulées en matière plastique, par exemple en résine synthétique ABS, dont la surface est au moins partiellement recouverte d'une couche conductrice électrique, par exemple une couche d'un métal tel que l'argent, le chrome, l'aluminium, formée ou appliquée par toute technique appropriée telle que la métallisation sous vide ou la pulvérisation thermique.

Deux sondes endonasales 8a et 8b, semblables à celle qui est représentée à la figure 5, traversent des alésages respectifs 85a et 85b ménagés dans les parties latérales 83a et 83b, respectivement, du support 81. Les parties médianes 30a et 30b des sondes 8a et 8b sont agencées de manière à pouvoir coulisser en glissant sur les parois intérieures des alésages 85a et 85b qui sont également recouvertes d'une couche conductrice électrique reliée électriquement à celle qui recouvre les parties 83a et 83b. La couche conductrice recouvrant la partie 82 est également en contact électrique avec celle qui recouvre les parties 83a et 83b. Ainsi, les impulsions de courant électrique ou d'ondes électromagnétiques provenant des générateurs d'énergie de l'appareil selon l'invention, par l'intermédiaire de la ligne de transmission 14 (figure 1) peuvent être transmises de la partie arrière 89 de la partie centrale 82, qui sert au raccordement électrique avec la ligne 14, jusqu'aux couches conductrices qui recouvrent les sondes 8a et 8b. Grâce à la rotation des parties latérales 83a et 83b, ainsi qu'au coulissement des sondes 8a et 8b dans les alésages 85a et 85b, les sondes 8a et 8b peuvent être mises en place avec une très grande précision de façon que leurs extrémités avant soient en contact avec les surfaces désirées des muqueuses endonasales, comme représenté à la figure 12 (c). En outre, comme représenté schématiquement en pointillé à cette figure, les sondes peuvent s'incurver grâce à leur flexibilité, de manière à épouser la forme du cavum endonasal.

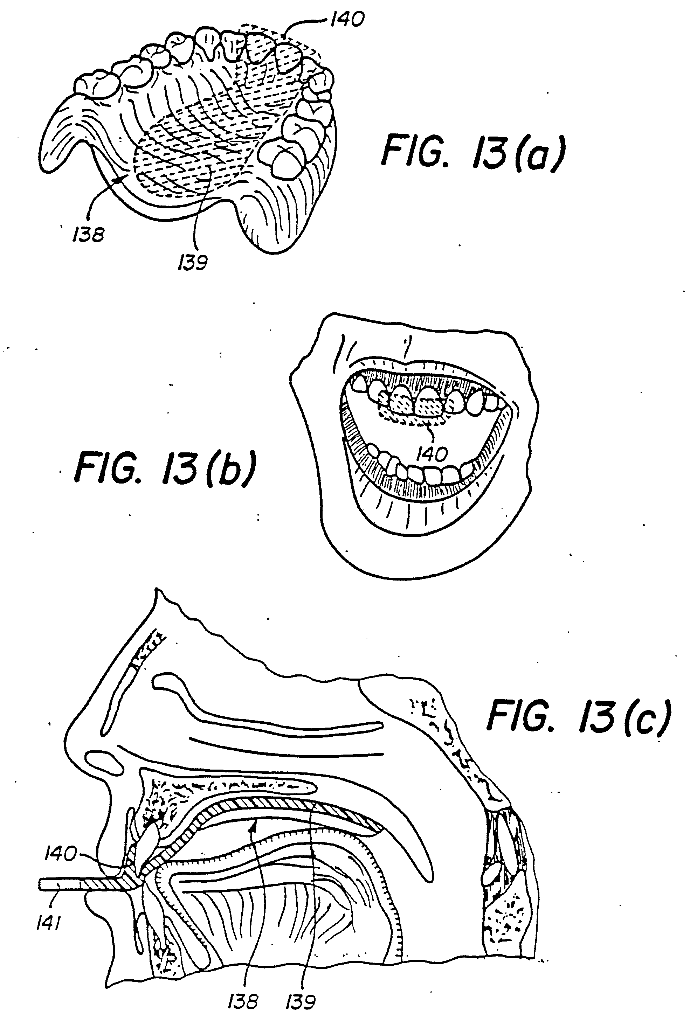

L'applicateur d'énergie endobuccal 138, représenté aux figures 13 (a), 13 (b) et 13 (c), a une forme en partie similaire à celle d'une prothèse dentaire. Sa partie centrale 139 est agencée de manière à pouvoir épouser la forme du palais, jusqu'à la terminaison arrière de la partie cartilagineuse du voile. Sa partie avant comprend une pince 140 agencée de manière à pouvoir être placée sur les incisives, de façon à permettre de fixer la position de l'applicateur 138. Celui-ci peut en outre être maintenu en place par la pression de la langue. La liaison électrique de l'applicateur d'énergie 138 avec la ligne de transmission d'énergie 14 (figure 1) est effectué par l'intermédiaire d'une partie de connexion 141, jouant le même rôle que la partie 79 de l'applicateur d'énergie illustré aux figures 7 à 11, la surface de l'applicateur 138 étant entièrement ou partiellement recouverte par une couche conductrice électrique.

En exerçant au moyen de l'appareil qui vient d'être décrit, des effets de stimulations successives par un signal constitué par une série d'impulsions rectangulaires de courant continu ayant une largeur d'impulsions de 2 millisecondes, avec une fréquence de 70 Hertz sous une tension de 0,03 Volts, la durée de chaque série d'impulsions étant de 15 secondes et un signal, modulé sinusoïdalement avec une fréquence de 50 Hertz, de courant alternatif à haute fréquence ayant une fréquence de 27 Mégahertz, avec une puissance de 500 milliwatts (tension alternative de sortie du signal modulé : 50 Volts), la durée d'application de ce deuxième signal étant de 25 secondes, ces deux types de stimulation pendant une durée totale de 5 à 20 minutes, on obtient une combinaison d'une action favorable sur l'équilibre nerveux, notamment sur l'équilibre neurovégétatif, résultant vraisemblablement de la combinaison de stimulations par les impulsions électriques rectangulaires de courant continu et par le courant alternatif à haute fréquence modulé par le signal à très basse fréquence.

## Revendications

1. Appareil pour le traitement thérapeutique de tissus vivants par stimulation au moyen d'impulsions de courant électrique et/ou d'ondes électromagnétiques, comprenant un générateur d'énergie électrique, caractérisé par le fait que le moyen pour appliquer l'énergie électrique au tissu à traiter est un applicateur endobuccal ou endonasal adapté pour transmettre l'énergie électrique reçue du générateur, que le générateur est adapté pour générer des impulsions de courant alternatif, à une fréquence variable de 20 à 100 megahertz, modulé avec une fréquence de modulation variable de 2,5 à 6 000 hertz, ou que le générateur est adapté pour générer des impulsions de courant continu de forme rectangulaire dont la durée d'impulsion est variable de 0,5 à 5 millisecondes, dont l'amplitude est variable de 10 à 100 millivolts et dont la fréquence de répétition est variable de 10 à 100 hertz.

2. Appareil selon la revendication 1, caractérisé par le fait que le moyen pour l'application de courant électrique et/ou d'ondes électromagnétiques est constitué par un applicateur endobuccal, spécialement conçu de manière à pouvoir épouser la forme du palais.

3. Appareil selon la revendication 2, caractérisé par le fait que l'applicateur endobuccal de courant électrique et/ou d'ondes électromagnétiques est pourvu d'une pince de fixation s'appliquant sur les incisives permettant ainsi le maintien de l'applicateur d'énergie dans la bouche du patient, en une position adéquate.

4. Appareil selon la revendication 1, caractérisé par le fait que le moyen pour l'application d'impulsions de courant électrique et/ou d'ondes électromagnétique est constitué par une sonde endonasale ayant la forme générale d'un stylet.

5. Appareil selon la revendication 4, caractérisé par le fait que ladite sonde est constituée par une seule pièce venue de moulage, en matière plastique, dont la surface est au moins en partie recouverte par une couche conductrice électrique, agencée de manière à permettre la transmission d'un courant électrique entre la partie arrière de la sonde et sa partie avant par l'intermédiaire de sa partie médiane.

## Claims

1. Device for the therapeutic treatment of living tissues by stimulation by means of electric current impulses and/or electromagnetic waves, comprising an electric energy generator, characterized in that the means for applying the electric energy to the tissue to be treated is an endobuccal or endonasal applicator adapted to transmit the electric energy received from the generator, in that the generator is adapted to generate impulses of alternating current, at a variable frequency of 20 to 200 megahertz, modulated with a variable modulation frequency of 2.5 to 6,000 hertz, or in that the generator is adapted to generate impulses of direct current of rectangular form whose impulse duration is variable from 0.5 to 5 milliseconds, whose amplitude is variable from 10 to 100 millivolts and whose recurrence frequency is variable from 10 to 100 hertz.

2. Device according to Claim 1, characterized in that the means for the application of electric current and/or electromagnetic waves is comprised of an endobuccal applicator, specially designed so as to be able to fit the shape of the palate.

3. Device according to Claim 2, characterized in that the endobuccal applicator of electric current and/or electromagnetic waves is provided with a fixing clip applied to the incisors, thus allowing the energy applicator to be held in the mouth of the patient, in a suitable position.

4. Device according to Claim 1, characterized in that the means for the application of electric current impulses and/or electromagnetic waves is comprised of an endonasal probe having the general form of a stylet.

5. Device according to Claim 4, characterized in that the said probe is comprised of a single integrally cast piece, of plastic material, whose surface is at least partly covered by an electric conducting coat arranged in such a manner as to permit the transmission of an electric current between the rear part of the proble and its front part by way of its middle part.

## Patentansprüche

1. Gerät zur therapeutischen Behandlung von lebendem Gewebe durch Stimulation mit elektrischen Stromimpulsen und/oder elektromagnetischen Wellen, mit einem elektrischen Energiegenerator, dadurch gekennzeichnet, daß die Einrichtung zum Applizieren der elektrischen Energie auf das zu behandelnde Gewebe ein innerhalb der Mundhöhle oder der Nase anzuordnender Applikator ist zum Übertragen der elektrischen Energie, die von dem Generator erhalten wird, daß der Generator ausgebildet ist zum Erzeugen von Wechselstromimpulsen mit einer variablen Frequenz von 20 bis 100 mHz, die mit einer variablen Modulationsfrequenz von 2,5 bis 6 000 Hz moduliert sind, oder daß der Generator ausgebildet ist zum Erzeugen von Gleichstromimpulsen mit rechteckiger Form, deren Impulsdauer variabel ist von 0,5 bis 5 msec, deren Amplitude variabel ist von 10 bis 100 mV und deren Grundfrequenz variabel ist von 10 bis 100 Hz.

2. Gerät nach Anspruch 1, dadurch gekennzeichnet, daß die Einrichtung zum Applizieren des elektrischen Stromes und/oder der elektromagnetischen Wellen aus einem endobukkalen oder in der Mundhöhle anzuordnenden Applikator besteht, der speziell ausgebildet ist, um sich der Form des Gaumens anpassen zu können.

3. Gerät nach Anspruch 2, dadurch gekennzeichnet, daß der endobukkale oder in der Mundhöhle anzuordenbare Applikator für den elektrischen Strom und/oder die elektromagnetischen Wellen in Form einer Befestigungsklammer ausgebildet ist, die an den Schneidezähnen angeordnet wird, wodurch das Festhalten des Energieapplikators in dem Mund des Patienten in einer geeigneten Stellung möglich ist.

4. Gerät nach Anspruch 1, dadurch gekennzeichnet, daß die Einrichtung zum Applizieren der elektrischen Stromimpulse und/oder der elektromagnetischen Wellen aus einer endonasalen oder in der Nase anordenbaren Sonde besteht, die im wesentlichen die Form eines Stiletts aufweist.

5. Gerät nach Anspruch 4, dadurch gekennzeichnet, daß die Sonde aus einem einzigen durch Gießen aus Kunststoffmaterial hergestellten Teil besteht, dessen Oberfläche wenigstens z. T. mit einer elektrisch leitenden Schicht bedeckt ist, die derart angeordnet ist, daß eine Übertragung eines elektrischen Stromes zwischen dem hinteren Abschnitt der Sonde und ihrem vorderen Abschnitt über ihren mittleren Abschnitt möglich ist.

FIG. 1

FIG. 2

FIG. 3

FIG. 4

1

FIG. 5

FIG. 6

FIG. 7

FIG. 8

FIG. 8 (b)

FIG. 8 (c)

FIG. 8 (d)

FIG. 11

FIG. 10

FIG. 9

FIG. 12 (a)

FIG. 12 (b)

FIG. 12 (c)

FIG. 13 (a)

FIG. 13 (b)

FIG. 13 (c)